# EUROPEAN PATENT APPLICATION

(11) **EP 2 005 841 A1**
(43) Date of publication of application: **24.12.2008**
(21) Application number: 07740670.0
(22) Date of filing: 30.03.2007
(51) Int. Cl.: A23L 1/30, A23K 1/16

(54) **COMPOSITION FOR BEVERAGE OR FOOD**

(30) Priority: 31.03.2006 JP 2006098901
(71) Applicant: Nippon Paper Chemicals Co., Ltd., Tokyo 1020076 (JP); Miyarisan Pharmaceutical Co., Ltd., Sakakimachi Hanishina-gun Nagano 389-0602 (JP)
(72) Inventor: TABATA, Masahiko, Tokyo 102-0076 (JP); TAKAHASHI, Motomichi, Tokyo 114-0016 (JP)
(74) Representative: Waldren, Robin Michael
(86) International application number: PCT/JP2007/057234
(87) International publication number: WO 2007/114378

(57) **Abstract**

It is an object of the present invention to provide a composition for a food or beverage containing a butyric acid bacterium which produces butyric acid and is excellent in intestinal regulation and cellooligosaccharide which serves as a substrate for the butyric acid bacterium and facilitates the production of butyric acid, enhancing an amount of butyric acid produced in intestine and being excellent in functions to improve an enteric environment and protect against an infectious disease. That is, the present invention provides a composition for a food or beverage containing a butyric acid bacterium and cellooligosaccharide and having a function to improve an enteric environment and/or a function to protect against an infectious disease, a composition for a food or beverage wherein the butyric acid bacterium is *Clostridium butyricum,* a feedstuff containing a butyric acid bacterium and cellooligosaccharide and having a function to improve an enteric environment and/or a function to protect against an infectious disease, and a food containing a butyric acid bacterium and cellooligosaccharide and having a function to improve an enteric environment and/or a function to protect against an infectious disease.

## Description

### TECHNICAL FIELD

The present invention relates to a composition for a food or beverage comprising a butyric acid bacterium and cellooligosaccharide. More particularly, the present invention relates to a composition for a food and beverage which contains the butyric acid bacterium which produces butyric acid and is excellent in intestinal regulation and also contains cellooligosaccharide which serves as a substrate for the butyric acid bacterium and facilitates the production of butyric acid. The present composition improves the amount of butyric acid produced in intestine and has excellent functions to improve an enteric environment and protect against infection. The present invention also relates to use thereof.

### BACKGROUND ART

The intestinal tract has not only roles of digesting and absorbing nutritional elements required for life maintenance but also roles of protecting a body against foreign enemies such as harmful bacteria and viruses, and foreign substances (intestinal immunity function), and is an very important organ for health maintenance. According to recent studies on its physiological functions, it has been elucidated that the improvement of the enteric environment including intestinal flora (bacterial flora) is greatly involved in prevention of diseases (prevention of gastrointestinal diseases, reduction of risk for carcinogenesis) and health maintenance and enhancement. It is known that 100 trillion cells of bacteria which are classified to 100 or more types are normally present in the intestinal tract, and they are broadly divided into beneficial bacteria such as *Bifidobacterium* and *Lactobacillus,* and putrefactive bacteria such as *Clostridium perfringens, Bacteroides* and *Escherichia coli.* As materials which assist the enteric bacteria to promote the growth of the beneficial bacteria and restrain the putrefactive bacteria, a variety of probiotics (living microbial preparations which serve profitably for host animals by improving a balance in the intestinal flora) and prebiotics (factors such as oligosaccharide and dietary fibers which grow the beneficial bacteria in the intestine) have been placed on the market.

As the substances which significantly grow the beneficial bacteria such as *Bifidobacterium* which inherently live in the body, the prebiotics is available, and a variety of oligosaccharides and dietary fibers are used therefor. Examples of the oligosaccharides may include nondigestible oligosaccharides such as fructooligosaccharide, isomaltooligosaccharide, galactooligosaccharide, xylooligosaccharide and raffinose oligosaccharide. The nondigestible oligosaccharide is not digested in stomach and small intestine, reaches the large intestine, and is assimilated by enteric bacteria in the large intestine to produce short chain fatty acids such as acetic acid, propionic acid, butyric acid and lactic acid. These short chain fatty acids cause physiological activities such as increase of a mucosal blood amount in gastrointestinal blood flow, growth of gastrointestinal mucosal epithelial cells and inhibition of cholesterol synthesis.

Among the short chain fatty acids, if lactic acid is accumulated excessively, it causes mucosal disorder and thus harmfully affects metabolism of large intestine epithelial cells. Meanwhile, as to butyric acid, a majority thereof is incorporated into the large intestine epithelial cells before coming in a systemic energy pool, and activates the metabolism. Butyric acid also has effects on DNA repair and prevention of large intestine cancer. Furthermore, acetic acid, propionic acid and butyric acid are not only involved in inhibiting the growth of the harmful bacteria by lowered pH in the intestine but also utilized as energy sources...These acids further have an effect of promoting a peristaltic motion of the intestine.

Among oligosaccharides, fructooligosaccharide which is the representative prebiotics is obtainable by linking fructose to succrose via 1,4-linkages. It has been said that, since fructooligosaccharide is selectively assimilated by enteric beneficial bacteria, particularly *Bifidobacterium* and has nondigestible property, fructooligosaccharide greatly contributes to the improvement of the intestinal bacterial flora. However, fructooligosaccharide is not sufficiently stable, and in particular when stored in an acidic solution for a long time, it is changed to glucose, fructose and sucrose, resulting in the reduced physiological activity. The other oligosaccharides have the same drawbacks. The growth of *Bifidobacterium* reduces putrefactive substances such as skatole and indole, but the short chain fatty acids produced by bifidobacterium are mainly lactic acid and acetic acid. Thus, when bifidobacterium becomes predominant in the bacterial flora in the large intestine, the concentration of butyric acid is undesirebly reduced. From such a context, oligosaccharides which are selectively assimilated by butyric acid bacteria have been desired.

When the putrefactive bacteria are predominant in the intestinal bacterial flora, the effect by the prebiotics alone is very small. Thus, in order to effectively increase the beneficial enteric bacteria, a synbiotics combining the living microbial preparation having a probiotics function with oligosaccharide having a prebiotics function has been commercialize.

JP 2003-93019-A (Patent Document 1) discloses a composition for oral ingestion containing Bifidobacterium as the probiotics and xylooligosaccharide as the prebiotics, and provides a composition for the oral ingestion which is compact and excellent in storage stability. JP 2005-34135-A (Patent Document 2) discloses a function-augmenting composition which improves bioavailability of a food active component comprising one or more of the probiotics, the prebiotics and biogenics. JP 2004-357505-A (Patent Document 3) discloses a supplement for preventing and ameliorating gastrointestinal diseases in dogs containing beer yeast, a living microbial preparation, oligosaccharide and a tea extract. In these prior art references, *Bifidobacterium* growth factors are focused. However, no one has focused on the production of butyric acid. Thus nothing which sufficiently satisfies the improvement of the enteric environment is available.

JP Hei-7-145 (Patent Document 4) discloses a butyric acid bacterium composition composed of butyric acid bacteria and glucomannan. Although glucomannan was assimilated by the butyric acid bacteria, the amount of produced butyric acid was not sufficient to improve the enteric environment.

Patent Document 1: JP 2003-93019-A
Patent Document 2: JP 2005-34135-A
Patent Document 3: JP 2004-357505-A
Patent Document 4: JP Hei-7-145

### DISCLOSURE OF INVENTION

### PROBLEM TO BE SOLVED BY THE INVENTION

It is an object of the present invention to provide a composition for a food or beverage capable of remarkably improving an enteric environment.

### MEANS FOR SOLVING PROBLEM

As a result of an extensive study to solve the aforementioned problem, the present inventors have found the following fact. That is, when butyric acid bacteria, which produce butyric acid and have excellent intestinal regulation activity among a variety of living microbial preparations, is combined with cellooligosaccharide, cellooligosaccharide can be served as a substrate for the butyric acid bacteria, which leads to a remarkable increase in the amount of butyric acid produced in intestine by butyric acid bacteria. They have also found that this combination can realize the improvement of an enteric environment through effective augmentation of beneficial enteric bacteria and reduction of the amount of putrefactive substances. The present inventors have completed the present invention based on such findings, and provide the following as the realization of the invention.

(1) A composition for a food or beverage comprising a butyric acid bacterium and cellooligosaccharide, the composition having a function to improve an enteric environment and/or a function to protect against an infectious disease.
(2) The composition for the food or beverage according to (1) wherein the butyric acid bacterium is *Clostridium butyricum.*
(3) A feedstuff comprising a butyric acid bacterium and cellooligosaccharide, the feedstuff having a function to improve an enteric environment and/or a function to protect against an infectious disease.
(4) A food comprising a butyric acid bacterium and cellooligosaccharide, the food having a function to improve an enteric environment and/or a function to protect against an infectious disease.
(5) Use of a butyric acid bacterium and cellooligosaccharide as a composition for a food or beverage having a function to improve an enteric environment and/or a function to protect against an infectious disease.
(6) Use of a butyric acid bacterium and cellooligosaccharide as a feedstuff or a food having a function to improve an enteric environment and/or a function to protect against an infectious disease.

### EFFECT OF THE INVENTION

The composition for the food or beverage of the present invention not only allows cellooligosaccharide to serve as the substrate of the butyric acid bacteria but also can remarkably enhance a capacity of the butyric acid bacteria to produce butyric acid in the intestine. Furthermore, it not only decreases the putrefactive bacteria and increases the beneficial bacteria, but also can improve the enteric environment through, e.g., decreasing the putrefactive substances. Therefore, administration of the composition for the food or beverage of the present invention to human beings and animals brings about increase of the amount of the butyric acid bacteria and butyric acid in the large intestine, which enables maintenance of large intestine epithelial cells in a healthy state. As a result, a protection capacity against a variety of infectious diseases is enhanced, leading to the prevention of large intestine cancer and large intestine diseases, and the health can be maintained and promoted. Also when the present composition is administered to livestock, a morbidity is reduced, days until shipping can be shortened because it is excellent in weight gain, which lead to cost reduction and economic benefit.

### BRIEF DESCRIPTION OF DRAWINGS

FIG. 1 is a graph showing flora in feces from piglets 30 days after weaning in Example 3.
FIG. 2 is a graph showing amounts of skatole (SKT) in feces from piglets 30 days after weaning in Example 3.
FIG. 3 is a graph showing organic acid compositions in feces from piglets 30 days after weaning in Example 3.
FIG. 4 is a graph showing amounts of ammonia in feces from piglets 30 days after weaning in Example 3.
FIG. 5 is a graph showing serum ammonia levels in feces from piglets 30 days after weaning in Example 3.
FIG. 6 is a graph showing organic acid compositions in appendix contents from neonatal chicks (18 days of age, and 42 days of age) in Example 4.

### BEST MODE FOR CARRYING OUT THE INVENTION

The composition for a food or beverage of the present invention contains butyric acid bacteria and cellooligosaccharide. That is, the composition for the food or beverage of the present invention contains the butyric acid bacteria as a probiotics and cellooligosaccharide as a prebiotics, and has effects to increase a butyric acid concentration and maintain a healthy state in the intestine, particularly in the large intestine. That is, the composition for the food or beverage of the present invention exerts in a living body both or at least either one of a function to improve an enteric environment and a function to protect against infectious diseases.

The first active component of the composition for the food or beverage of the present invention is butyric acid bacteria. Generally, *Bifidobacterium* (*Bifidobacterium* sp.) and *Lactobacillus* (*Lactobacillus* sp.) are used as the probiotics. Beneficial bacteria such as *Bificobacterium* are predominant in intestinal flora in infants. However, with aging, the beneficial bacteria decrease and a ratio of putrefactive bacteria increases. Thus, for the purpose of restrain the putrefactive bacteria and increasing the amount of beneficial bacteria, many types of living microbial preparations such as *Bifidobacterium* and *Lactobacillus* are used as the probiotics. However, orally ingested *Bifidobacterium* and *Lactobacillus* are not resistant to acids such as gastric juice and bile, and only a very small percentage thereof is taken alive to the intestine. Even when a part thereof settles in the intestine, discontinuation of its ingestion will again cause predominance of the putrefactive bacteria in the intestinal flora. On the contrary, the butyric acid bacteria used in the present invention form spores under an aerobic environment, and after being orally administered, they are exposed to a low pH environment due to a protein digestive juice pepsin and the gastric juice. The resistant butyric acid bacteria as the spores pass through here without being eradicated, and reach duodenum. There, pH is elevated to around a neutral, the foods are digested with a variety of digestive juices, the nutritious environment is formed, and then, the butyric acid bacteria acquire a chance to germinate and grow. Thus, the butyric acid bacteria are not digested with the gastric juice and the bile even when orally ingested, and can reach the large intestine as the spores. Thereby the butyric acid bacteria can germinate and grow to produce butyric acid in the large intestine.

The butyric acid bacteria used in the present invention may be any bacterial species as long as they produce butyric acid. Among them, the bacterial species belonging to genus *Clostridium* is preferable, and *butyricum* species is particularly preferable. Furthermore, the typical and preferable strain belonging to *Clostridium butyricum* may be *Clostridium butyricum* Miyairi strain. *Clostridium butyricum* is an obligate anaerobe and forms the spore. This strain was reported as the butyric acid bacterium isolated from human intestine and having a strong antagonistic action upon the putrefactive bacteria by Doctor Kinji Miyairi, Department of Hygiene, Chiba Medical Collage (now belongs to the Chiba University, School of Medicine) in 1933. This bacterial strain has an antagonistic action against a variety of gastrointestinal pathogens including the putrefactive bacteria and exerts an intestinal regulation effect by coexisting with so-called enteric beneficial bacteria such as *Bifidobacterium* and *Lactobacillus.* It has been also reported that this bacterial strain is more stable in formulations and more resistant to the gastric juice than a *Lactobacillus* group.

The butyric acid bactera used in the present invention may include specifically *Lactobacillus plantarum, Clostridium butyricum* NIP1006, *Clostridium butyricum* NIP1015, *Clostridium butyricum* NIP1017 and *Clostridium butyricum* Miyairi 588. Among them, *Clostridium butyricum* Miyairi 588 having a high capacity to produce butyric acid is preferable. *Clostridium butyricum* Miyairi 588 was deposited to Ministry of International Trade and Industry, Agency of Industrial Science and Technology, Fermentation Research Institute (1-1-3 Higashi, Tsukuba-shi, Ibaraki Prefecture, Japan, Postal code 305)[now International Patent Organism Depositary, National Institute of Advanced Industrial Science and Technology (Central No. 6, 1-1-1 Higashi, Tsukuba-shi, Ibaraki Prefecture, Japan 305-8566) as of May 1, 1981, and its depository number is FERM BP-2789 (transferred from Fermentation Research Institute, deposited bacterium P 1467 deposited on May 16, 1972).

As the butyric acid bacterium for the composition for the food or beverage of the present invention, a commercially available living microbial preparation such as Miyarisan tablet (supplied from Miyarisan Pharmaceutical Co., Ltd.) may be used. Alternatively, the butyric acid bacteria may be cultured in an appropriate liquid medium and microbial cells may be isolated and directly used, or dried and used as dry microbial cells. When the commercially available living microbial preparation is used, the adding amount is adjusted considering the purity and a content of butyric acid bacteria therein so that the resulting composition contains the following number of the bacteria.

In the composition for the food or beverage of the present invention, cellooligosaccharide is used as a second active component. Cellooligosaccharide is assimilated particularly selectively by the enteric butyric acid bacteria and greatly contributes to the growth of the butyric acid bacteria, thus leading to increase of larger amount of butyric acid production than fructooligosaccharide. In addition, cellooligosaccharide is highly stable under an acidic condition. Therefore, the cellooligosaccharide effectively acts as the prebiotics in the composition for the food or beverage of the present invention.

In the present invention, cellooligosaccharide is an oligosaccharide in which two or more glucoses is linked via 1,4-linkages. Cellooligosaccharide is usually a mixture of oligosaccharides having a variety of polymerization degrees, but may be purified to have a single polymerization degree or have a polymerization degree within a particular range. Among the aforementioned oligosaccharides, it is preferable in the present invention to abundantly contain at least one of cellobiose, cellotriose, cellotetrose, cellopentaose and cellohexaose having a glucose polymerization degree of 2 to 6. Particularly it is preferable to abundantly contain at least one of cellobiose, cellotriose and cellotetrose, and it is further preferable to abundantly contain at least one of cellobiose and cellotriose. Specifically, it is preferable that the content of cellooligosaccharide having the glucose polymerization degree of 2 to 6 is 50% by weight or more, preferebly 80% by weight or more and particularly preferably 90% by weight or more. It is further desirable that the content of cellobiose is 70% by weight or more, preferably 85% by weight or more, more preferably 90% by weight or more, and still more preferably 95% by weight or more. The steric isomerism of cellooligosaccharide is not particularly limited, but it is generally a D-isomer.

Cellooligosaccharide for use in the present invention may be produced by the publicly known method. An example of a known chemical method therefor is a method in which cellulose is acid-hydrolyzed with fuming hydrochloric acid/concentrated sulfuric acid, and then the resulting cellooligosaccharide is fractionated by a carbon column (Miller, G. L, Methods in Carbohydrate Chemistry III (Academic Press), 134 (1963)).

Examples of known enzymatic methods therefor may include a method in which cellulase produced by a microorganism belonging to genus *Cellvibrio* is allowed to act upon amorphous cellulose, while a product inhibition is suppressed by a concurrently used ultrafiltration reactor, to synthesize cellooligosaccharide (see JP Hei-1-256394-A); a method of producing cellooligosaccharide by allowing cellulase from which β-glycosidase has been selectively removed to act upon cellulose (see JP Hei-5-115293-A); and a method of producinng cellooligosaccharide containing cellobiose in a system for allowing cellulase to act upon unbleached sulfite pulp in a wet state as a raw material, combined with an ultrafiltration reactor (JP Hei-8-2312-B).
There is also known another method in which a reverse reaction of cellodextrin phosphorylase is utilized, and cellooligosaccharide is produced in the presence of cellobiose with the use of glucose-1-phosphate as a glucose donor (Journal of Fermentation and Bioengineering, vol. 77, No.3, 239-242 (1994)).

As cellooligosaccharide in the composition for the food or beverage of the present invention, commercially available products (e.g., supplied from CMS Chemicals) may also be used in addition to the products produced by any of the aforementioned methods. In the present invention, suitable method for cellooligosaccharide production is a method in which cellulose is degraded into cellooligosaccharide using cellulase and the purity of cellooligosaccharide having a glucose polymerization degree of 2 to 4 is elevated through a crystallization process.

Furthermore, cellooligosaccharide not only serves as the substrate for the butyric acid bacteria but also has other inherent physiological actions. For example, as to its effect on lipid metabolism, it has been reported that, when rats were fed with cellobiose-added high sucrose food for 4 weeks, a body fat ratio was reduced and levels of total cholesterol and neutral fat were also reduced compared with control rats (Takashi Watanabe, Cellulose Commun., 5, 91 (1998)). It has been also reported that an enzymatic activity for fatty acid synthesis is inhibited whereas an enzymatic activity for fatty acid degradation is increased by giving a cellobiose-added feedstuff to broiler chickens or laying hens (Aiko Ishida, Hitoshi Murakami, Makoto Yamazaki, Makoto Otsuka, Katsuhiro Mamoto, Hideya Honma, Yukio Kanai and Ryozo Takada, Proceedings for the 103rd Meeting of Japanese Society of Animal Science, 52 (2004); Aiko Ishida, Makoto Otsuka, Masaya Katsumata, Ryozo Takada and Yukio Kanai, Proceedings for the 104th Meeting of Japanese Society of Animal Science, 69 (2005)). This way, it is conceivable that cellobiose not only serves as the substrate for the butyric acid bacteria to exert the effect on the butyric acid production but also itself favorably affects the lipid metabolism in vivo to help the prevention of lifestyle-related diseases. It is speculated that the excellent physiological actions inherent in cellobiose are also exerted simultaneously in the composition for the food or beverage of the present invention.

The present inventors also have found out that, among a variety of enteric bacteria, the bacteria belonging to genus *Clostridium* which are the butyric acid bacteria can assimilate cellooligosaccharide well, and that *Clostridium butyricum* can particularly assimilate cellooligosaccharide well and have high butyric acid productivity.

In order to observe whether the butyric acid bacteria actually grow in a selective manner when cellooligosaccharide is added to human feces, a feces suspension was prepared, corn starch or cellooligosaccharide was added thereto, the butyric acid bacteria were inoculated thereto, and the resulting mixture was cultured. As a result, it was found out that pH remarkably was lowered and the butyric acid bacteria grew in a cellooligosaccharide-added suspension compared with a control corn starch-added suspension.
Furthermore, the butyric acid bacteria were given to the rats which had ingested cellooligosaccharide ad libitum for 3 days and the number of the butyric acid bacteria in their enteric content was examined. As a result, it was found out that the number of the enteric butyric acid bacteria in the rat which had ingested cellooligosaccharide was larger than that in the rats which had not ingested cellooligosaccharide.

These experimental results suggest that cellooligosaccharide is not assimilated by indigenous intestinal bacteria and is selectively assimilated only by the concurrently administered butyric acid bacteria in the gastrointestinal tract. Thus it can be said that the composition combining cellooligosaccharide and the butyric acid bacteria like the present invention leads to the increase of the bacterial number thereof.

The amounts of the butyric acid bacteria and cellooligosaccharide in the composition for the food or beverage of the present invention may be appropriately increased or decreased depending on an age and a disease symptom of a patient if used for medical care, or depending on each purpose if used for functional foods or animals.
The amount of the butyric acid bacteria is not particularly limited, and is typically in the range of 1×10⁵ to 1×10¹⁰ CFU (colony forming unit) and preferably 1×10⁶ to 1×10⁸ CFU as the bacterial number per day. The amount of the butyric acid bacteria in the composition for the food or beverage of the present invention may be appropriately adjusted based on this dosage with regard to a daily administration frequency.

Meanwhile, the amount of cellooligosaccharide to be added may be in the range of the amount required for serving as the substrate for the butyric acid bacteria and facilitating the intestinal regulation as the prebiotics. However, it is necessary to determine the amount in consideration of possible soft feces caused by the excessive ingestion of cellooligosaccharide. A maximum intake of cellooligosaccharide per day is preferably about 0.36 g per kg of body weight. Usually, the composition for the food or beverage of the present invention may contain 0.1 to 50% by weight, and preferably 1.0 to 30% by weight of cellooligosaccharide.
As to the administration method, the composition for the food or beverage of the present invention as a pharmaceutical is preferably taken once to three times daily after a meal, but may also be taken arbitrary. The composition as the functional food or for animals may be ingested freely, e.g., once to several times daily.

The composition for the food or beverage of the present invention may be produced, for example, by the following method.
The butyric acid bacteria cultured in publicly known CS medium (see JP Sho-59-187784-A) are separated from the medium to yield a bacterial paste by cebtrifugation. The bacterial past is dried to yield dry bacterial powder. Cellooligosaccharide is added to the dry bacterial powder, and the mixture is then kneaded by a kneader until it becomes uniform. Subsequently, vacuum drying is performed. The conditions for the vacuum drying may be specifically the conditions at 50°C or below for 5 hours at 10 mmHg using a shelf-type vacuum dryer. The resulting dried product was pulverized by a pulverizer to yield the composition. In this way, it is possible to obtain a milk-white, uniform and fine-granular composition having almost no taste and no odor.

The composition for the food or beverage of the present invention is applicable to all animals including human being. The present composition can be used for the animals at any age from the juvenile to the elderly without any limitation. The use thereof is not particularly limited by a health state and a physical size. Examples of the animals other than human being may include experimental animals such as rats, mice and rabbits, farm animals such as swines, cattle, sheeps, goats and horses, farm birds such as chickens, ducks, quails and turkeys, and pet animals such as cats and dogs.
The method for administering the composition for the food or beverage of the present invention is not particularly limited. The butyric acid bacteria and cellooligosaccharide may be mixed to configure pills or tablets which may be orally administered. It is also possible to produce powders or granules which are added to the food or beverage for the administration. Alternatively, the butyric acid bacteria and cellooligosaccharide may be formulated as distinct powders or formulations which may be added or utilized simultaneously upon the administration.

As a dosage form when the composition for the food or beverage of the present invention is used as the pharmaceutical, the powders of the components may be directly used without formulation, but it is possible to formulate the components in a form of powders, granules, fine granules, pills, sugar-coated tablets, capsules, tablets and enteric coating agents. An excipient, a binder and a disintegrant generally used for pharmaceutical formulations may be used as a diluent. In addition thereto, coloring agents, flavoring agents, stabilizers, preservatives and lubricants may also be added.

When the composition for the food or beverage of the present invention is used as the food, the powders of each components may be directly used without formulation, but it is possible to formulate the components to be in a form suitable for eating by adding other dietary fibers, oligosaccharides, grain crops and vitamins and further add flavors, coloring agents and flavoring agents. Also, the present composition may be used as a food additive by admixing the composition with another food.

As the feedstuff for the animals, the butyric acid bacteria and cellooligosaccharide may be mixed and used in the powder form without formulation, but it is also possible to add thereto a variety of excipients and additives in the same way as in the case for the food. The formulation may be in a form of powders or may be in any of other dosage forms. It is also possible to admix the components with maize powders, soybean cakes, barley powders, naked barley powders, soybean powders, rice bran, defatted rice bran, chaffs, potato powders, sugar cane powders, soybean curd cakes, starch, yeast and fish powders which are feedstuff ingredients.

### EXAMPLES

The present invention will be described in detail hereinbelow with reference to Examples, but the present invention is not limited thereto.

### [Method for measuring butyric acid]

350 µL Of distilled water was added to 150 mg of an appendix content, which was then homogenized using Polytron^{R} (supplied from Kinematica) (Lebel 30, 30 seconds, 4°C). The resulting homogenate was centrifuged (15,000 rpm, 15 minutes, 4°C) to yield a supernatant. 0.5 mL Of chloroform was added to 0.5 mL of the supernatant, which was then stirred for one minute using a Vortex^{R} mixer and centrifuged (15,000 rpm, 15 minutes, 4°C) to yield a supernatant. This supernatant was filtrated through a Millipore^{R} filter (supplied from Millipore), and 10 µL thereof was applied to HPLC.

### [HPLC condition]

Solution delivery system: Shimadzu LC-10AD (supplied from Shimadzu Corporation)
Column: Shim-pack SCR-102(H)
Detector: CDD-10A
Mobile phase: 5 mM p-toluenesulfonic acid
Reaction phase: 20 mM Bis-Tris (containing 5 mM p-toluenesulfonic acid and 100 mM EDTA)
Temperature: 45°C

### [Example 1]

Cellooligosaccharide (cellobiose 96% by weight, glucose 2% by weight, cellotriose 2% by weight) was added at 5% by weight to drinking water, and this was given ad libitum to ICR strain mice (body weight: about 30 g) for 3 days. Then, *Clostridium butyricum* Miyairi 588 (FERM BP-2789) was orally administered at 1.0×10⁸ bacteria per mouse. The mice were sacrificed 3 and 6 hours after the administration. Total gastrointestinal tract was removed, and the number of viable bacteria of *Clostridium butyricum* Miyairi in the content was counted in an anaerobic globe box using GAM plate medium. As controls, the same manipulation was performed in the mice which had not ingested cellooligosaccharide. Results are shown in Table 1.

**Table 1 Change in number of bacteria**

| Time elapsed | Group fed without cellooligosaccharide (Number of animals:6) | Group fed with Cellooligosaccharide (Number of animals: 6) |
|---|---|---|
| 3 hours | 2.1 x 10³ | 3.1 x 10⁴ |
| 6 hours | 3.8 x 10³ | 1.2 x 10⁴ |

As is found from Table 1, the amount of bacteria observed in the animals which had ingested cellooligosaccharide was larger than the amount in the controls. The increasing ratio of the bacterial number 3 hours after the administration was particularly high.

### [Example 2]

Wistar strain male rats (7 weeks of age) were used. The rats were pre-bred using a standard feedstuff containing corn starch as a carbohydrate source for 7 days to adapt to an experimental environment. Individuals having no abnormality were selected and 8 rats per one group were subjected to the experiment. Using the feedstuffs having the composition described in Table 2, the study was performed with a group fed with a cellooligosaccharide (cellobiose 96% by weight, glucose 2% by weight, cellotriose 2% by weight) (9% by weight) alone, and another group fed with a combination of butyric acid bacteria and cellooligosaccharide (cellooligosaccharide at 9% by weight plus butyric acid bacterium powders at 0.03% by weight, 0.1% by weight, or 0.3% by weight). As the butyric acid bacterium, *Clostridium butyricum* Miyairi 588 (FERM BP-2789) was used. The number of the butyric acid bacteria in the butyric acid bacterium powders was 4.1×10¹⁰ CFU/g. The blanc group was fed with a standard feedstuff (see the control in Table 2). The feedstuff was ingested ad libitum. The rats were bred for 14 days, adn then sacrificed. The content of butyric acid in the appendix content was measured using HPLC. For the ingestion of the butyric acid bacteria, the group in which the butyric acid bacterium powders were forcibly administered into their stomach once daily (dosage was 6.2×10⁹ CFU/200 g body weight) was examined in addition to the group of ad libitum digestion of the composition-containing feedstuff. To the feedstuff of the forcibly administered group, cellooligosaccharide at 9% by weight was added. The results of the butyric acid contents in the appendix content in each group are shown in Table 3.

**Table 2 Feedstuff composition (Numerals in the table are the contents (g) of each ingredient in 1 kg of feedstuff)**

| Composition | Control (Standard feedstuff) | Fed with cellooligosaccharide only | Fed with combination of butyric acid bacteria and cellooligosaccharide | | | |
|---|---|---|---|---|---|---|
| | | | Butyric acid 0.03wt% added | Butyric acid 0.1wt% added | Butyric acid 0. 3wt% added | Butyric acid forcibly administerd into stomach |
| Corn starch | 652.5 | 562.5 | 562.2 | 561.5 | 559.5 | 562.5 |
| Casein | 250.0 | 250.0 | 250.0 | 250.0 | 250.0 | 250.0 |
| Cellooligosaccharide | 0 | 90.0 | 90.0 | 90.0 | 90.0 | 90.0 |
| Butyric acid bacterium powders | 0 | 0 | 0.3 | 1.0 | 3.0 | 0 |
| Corn oil | 50.0 | 50.0 | 50.0 | 50.0 | 50.0 | 50.0 |
| Mineral mixture* | 35.0 | 35.0 | 35.0 | 35.0 | 35.0 | 35.0 |
| Vitamin mixture* | 10.0 | 10.0 | 10.0 | 10.0 | 10.0 | 10.0 |
| Choline bitartrate | 2.5 | 2.5 | 2.5 | 2.5 | 2.5 | 2.5 |

Note for Table 2: * A vitamin mixture and a mineral mixture were prepared in accordance with AIN-76 composition (Journal of Nutrition 107, 1340 (1977)).

**Table 3 Content of butyric acid**

| Groups | Control (Standard feedstuff) | Fed with cellooligosaccharide only | Fed with combination of butyric acid bacteria and cellooligosaccharide | | | |
|---|---|---|---|---|---|---|
| | | | Butyric acid 0.03wt% added | Butyric acid 0.1wt% added | Butyric acid 0. 3wt% added | Butyric acid forcibly administerd into stomach |
| Content of butyric acid (µ mol/g) | 4.5 | 12.0 | 15.5 | 15.0 | 14.0 | 17.0 |

From the results of this Example, it has been found that butyric acid was synergistically produced by combining cellooligosaccharide with the butyric acid bacteria, and that the synergistic effect was further enhanced by changing the administration of the butyric acid bacteria from the mixed feedstuff to the forced administration in the stomach. In particular, considering in conjunction with the results of Reference Example, it was found out the increased amount of the butyric acid bacteria in this Example increased the butyric acid content more noticeably than the increased amount of cellooligosaccharide. Therefore, the synergistic effect is exerted by combining cellooligosaccharide with the butyric acid bacteria to noticeably increase the butyric acid content in the intestine.

### [Reference Example 1]

The capacity of Miyairi bacterial strain (butyric acid bacterium) to assimilate cellooligosaccharide was compared with that of other enteric bacteria.
Bacterial strains tested were four strains i.e., Miyairi bacterial strain (*Clostridium butyricum* MIYAIRI 588(FERM BP-2789), *Bacillus subtilis* JCM 2499, Bifidobacterium adolescentis JCM1275 and *Lactobacillus casei* JCM1134.

The medium used for the test was the medium (PYC medium) prepared by adding cellooligosaccharide (cellobiose 96% by weight, glucose 2% by weight, cellotriose 2% by weight) at 1% by weight to the following basic medium and adjusting pH to 7.0.

(Basic medium) PY medium

| | |
|---|---|
| Peptone | 0.5 g |
| Trypticase | 0.5 g |
| Yeast extract | 1.0 g |
| Salts solution | 4.0 ml |
| Distilled water | 100.0 ml |
| Hemin solution | 1.0 ml |
| Vitamin K₁ | 0.02 ml |
| Cysteine HCl-H₂O | 0.05 g |

Each precultured bacterial strain was inoculated to the PYC medium so that an initial number of the bacteria was about 10⁵ CFU/mL. As the controls, each bacterial strain was inoculated to the PY medium. Miyairi bacterial strain, *Bifidobacterium* and *Lactobacillus* were cultured under an anaerobic condition, and *Bacillus subtilis* was cultured under an aerobic condition and under the anaerobic condition. The culture temperature was 37°C. Preculturing of each bacterial strain was performed as follows. Miyairi bacterial strain was inoculated to GAM broth and then cultured at 37°C for 6 hours. *Bacillus subtilis, Bifidobacterium* and *Lactobacillus* were inoculated to GAM broth and cultured at 37°C for 16 hours.

The ability of each bacterial strain to assimilate cellooligosaccharide was compared by the amount of organic acids produced when cellooligosaccharide was metabolized. Samples were collected 0, 6, 12, 24 and 48 hours after starting the culturing and the levels of the organic acids were measured by HPLC. The amount of the produced organic acid was calculated by subtracting, from the amount of the organic acids in the PYC medium, the amount of the organic acids in the PY medium at the same culturing period. The amounts (mM) of the organic acids produced by each bacterial strain for a certain time period (6, 12, 24 and 48 hours) after starting the culture were shown in Table 4.

**Table 4. Amount of produced organic acids**

| | Cultuirng time | Organic acids (mM) | | | | |
|---|---|---|---|---|---|---|
| | | Lactic acid | Formic acid | Acetic acid | Propion ic acid | Butyric acid |
| Miyairi bacteria | 6 h | 1.16 | 0.64 | 2.88 | 0.11 | 2.93 |
| | 12 h | 1.78 | 0.81 | 10.21 | 0.05 | 12.92 |
| | 24 h | ND | 0.66 | 12.45 | ND | 19.10 |
| | 48 h | ND | 0.79 | 12.52 | ND | 19.94 |
| *Batillus subtilis* (aerobic) | 6 h | 2.07 | ND | ND | 0.01 | ND |
| | 12 h | 0.39 | ND | ND | 0.02 | ND |
| | 24 h | 0.19 | ND | ND | 0.53 | ND |
| | 48 h | 0.23 | ND | 16.91 | 0.52 | ND |
| *Batillus subtilis* (anaerobic) | 6 h | ND | 0.31 | ND | ND | ND |
| | 12 h | ND | 0.33 | 0.02 | 0.01 | ND |
| | 24 h | ND | 0.37 | ND | ND | ND |
| | 48 h | ND | 0.37 | ND | ND | ND |
| *Bifidobacterium* | 6 h | ND | 0.18 | 0.11 | ND | ND |
| | 12 h | 1.01 | 0.57 | 2.58 | ND | ND |
| | 24 h | 1.08 | 0.73 | 2.97 | ND | ND |
| | 48 h | 1.08 | 1.08 | 3.64 | ND | ND |
| *Lactiobacillus* | 6 h | ND | 0.17 | ND | ND | ND |
| | 12 h | 0.21 | 0.15 | ND | ND | ND |
| | 24 h | 4.85 | 0.16 | ND | ND | ND |
| | 48 h | 26.43 | 0.29 | ND | ND | ND |

As is evident from Table 4, the Miyairi bacterial strain produced larger amount of the organic acids in earlier time than other bacterial strains. Only the Miyairi bacterial strain produced butyric acid.

### [Example 3]

For the efficacy of the present invention for pigs, the following parameters were examined and evaluated.
Three mother pigs per group were tested for a control group and an administration group (MGC group) of the present composition. Animals were kept in group housing. The samples were collected from each ten piglets among the piglets born from each mother pig.

The test was started as soon as obtaining the mother pigs at a late stage in the pregnancy. A test period was from just before childbirth to 30 days after weaning, and the sample was collected after the weaning and on a final day of the test (30 days after the weaning).
The composition of the feedstuff to be administered was controlled as follows depending on the growth of the piglets.

(Feedstuff to be administered)
a) For the piglets in a lactation period: the feedstuff prepared by adding the present composition at 0.5% by weight to a basic feedstuff (milk) was given until the weaning.
b) For the piglets after the weaning: the feedstuff prepared by adding the present composition at 0.2% by weight to a basic feedstuff was given for about 30 days.
c) For the mother pigs: the feedstuff prepared by adding the present composition at 0.2% by weight to a basic feedstuff was given.

### (Present composition)

- Ingredients:
   Butyric acid bacterium powders: 10% by weight
   Cellooligosaccharide: 20% by weight
   Starch: 20% by weight
   Glucose: 20% by weight
   Aluminium silicate: 20% by weight
   Calcium carbonate: 10% by weight
   As cellooligosaccharide, the composition of cellobiose 96% by weight, glucose 2% by weight and cellotriose 2% by weight was used.
   As the butyric acid bacterium powders, the Miyairi bacterial strain (*Clostridium butyricum* Miyairi 588 FERM BP-2789) was used so that the contained viable bacteria (spores) thereof were 10⁷ bacteria or more per one gram of the present composition.

The pigs (mother pigs and piglets) in the control groups were bred by administering the basic feedstuff without adding anything. The basic feedstuffs were commercially available feedstuffs containing antibiotics (avilamycin, colistin sulfate, morantel citrate). The commercially available feedstuffs mean the following feedstuff mixtures in the case of the piglets.

### (Feedstuff mixtures)

Lactation period: "Maruchu brand feedstuff mixture Primer I for piglet growth in sucking period "(supplied from Chubu Shiryo Co., Ltd.)
Lactation period to weaning period: "Maruchu brand feedstuff mixture Pro II for piglet growth in sucking period" (supplied from Chubu Shiryo Co., Ltd.)
After weaning: "Maruchu brand feedstuff mixture Welcome II for piglet growth in sucking period (supplied from Chubu Shiryo Co., Ltd.).

As to the feces from the piglets 30 days after the weaning in the MGC group and the control group, the amounts of flora, the organic acids and skatole (SKT) in the feces were measured. The amount of ammonia in the feces and the serum were also measured.

### [Flora in feces]

The bacterial numbers of the Miyairi bacterial strain (*Clostridium butyricum*), *C. perfringens, Enterobacteriaceae, Enterococcus* and *Lactobacillus* in the feces were measured as follows.

The feces collected from the piglets 30 days after the weaning in the control group and the MGC group was placed in a transport medium and suspended therein.
The suspension was serially diluted to 100 times using a diluent, and then smeared on a variety of selection media. For measuring the bacterial number, MIM medium was used for the Miyairi bacterial strain, NN medium was used for C. *perfringens,* and DHL medium was used for *Enterobacteriaceae.* TATAC medium was used for Enterococcus, and modified LBS medium was used for *Lactobacillus.* In the case of the DHL medium, the culturing was performed for one day. In the cases of the TATAC medium, the TS medium, the MIM medium and the NN medium, the culturing was performed for two days. In the cases of the BL medium and the LBS medium, the culturing was performed for three days. Formed colonies were counted to calculate the bacterial number (log CFU/g). For *Escherichia coli* among *Enterobacteriaceae,* pale pink colonies formed on the DHL medium were counted as the bacterial number.

Furthermore, among the enteric bacteria, the bacterial number of *Escherichia coli* toxin-producing strains was also measured. The *Escherichia coli* toxin-producing strain was detected by detecting the toxin produced by *Escherichia coli* on the DHL medium using VTEC-RPLA (Denka Seiken Co., Ltd.).

Experimental results were represented by mean values and standard deviations thereof of measured values of the individuals from each test group. Significance tests between the groups were performed using Student's t-test and Mann-Whitney U-test. A ratio of the number of the individuals in which each bacterial species had been detected to the number of the individuals which composed each test group was calculated as a detection ratio.

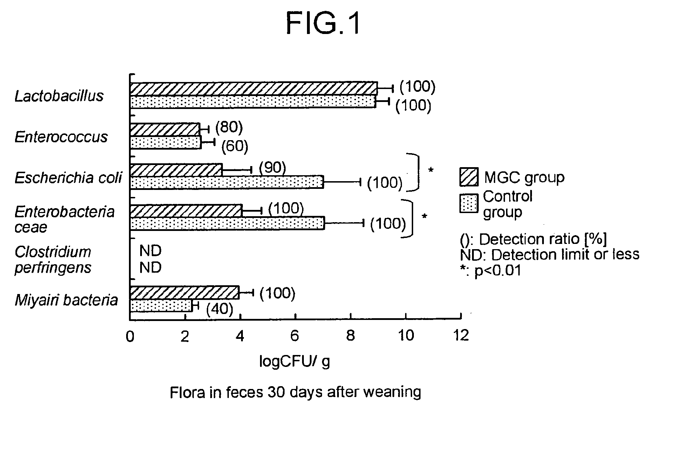

The same measurement and test were performed as to the feces of the control group 30 days after the weaning. The flora in the feces 30 days after the weaning in the MGC group and the control group are shown in FIG. 1.

As shown in FIG. 1, the Miyairi bacterial strain was detected in four specimens in the control group (2.3 ± 0.22 log CFU/g in the control group versus 4.0 ± 0.55 log CFU/g in the MGC group). *C. perfringens* was detected in none of the control and the MGC groups. The bacterial numbers of Enterobacteriaceae and *Escherichia coli* were significantly lower in the MGC group (*Enterobacteriaceae:* 7.1 ± 1.38 log CFU/g in the control group versus 4.1 ± 0.73 log CFU/g in the MGC group; *Escherichia coli*: 7.0 ± 1.39 log CFU/g in the control group versus 3.4 ± 1.04 log CFU/g in the MGC group). The bacterial numbers of *Enterococcus* and *Lactobacillus* were not different (Enterococcus: 2.6 ± 0.50 log CFU/g in the control group versus 2.6 ± 0.35 log CFU/g in the MGC group; *Lactobacillus*: 8.9 ± 0.51 log CFU/g in the control group versus 9.0 ± 0.59 log CFU/g in the MGC group). All of *Escherichia coli* strains isolated here from all strains were identified to be non toxin productive.

### [SKT in feces]

The feces 30 days after the weaning in the control group and the MGC group was diluted to 4 times with 0.03 M phosphate buffer (pH 7.4), and 0.3 mL thereof is dispensed in another tube for measuring SKT. An equal amount of acetonitrile was added thereto. The mixture was mixed well and left stand at -20°C for 15 minutes. The mixture was centrifuged at 6,000 rpm for 15 minutes, a supernatant was filtrated through a filter of 0.45 µL (brand name: Minisart supplied from Sartorius), and then applied to HPLC. The HPLC conditions are as follows.

### (HPLC condition)

Shimadzu Corporation LC-10 series
Column: Phennomenex ODS-C18
Column temperature: 40°C
Mobile Phase: 0.02 M Acetic acid 60v/v%
2-Propanol 15v/v%
Acetonitrile 25v/v%
Flow rate: 1 mL/minute
Detector: Ultraviolet spectrometer (Shimadzu Corporation) Wavelength: 280 nm
Analysis time period: 15 minutes

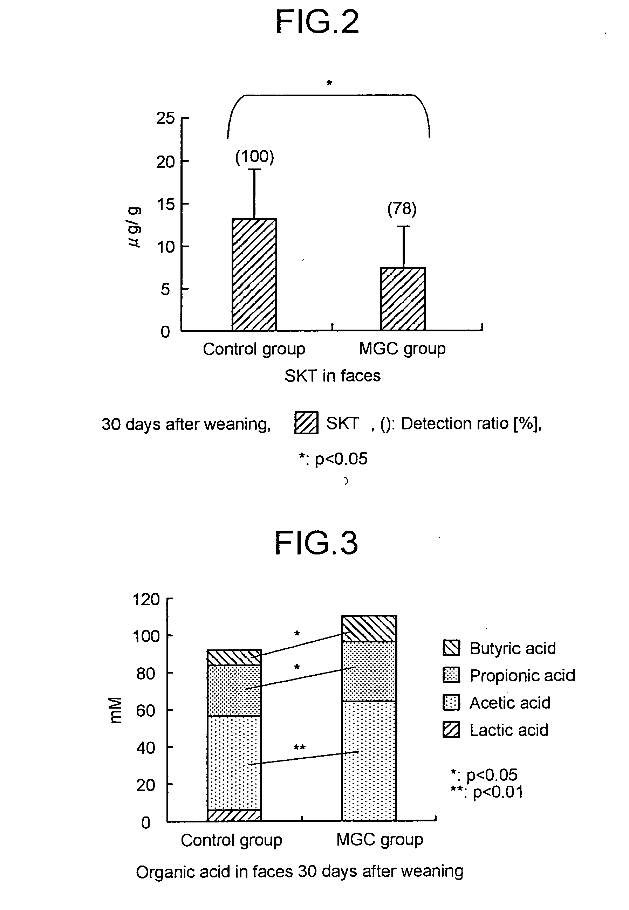

The amount (µg/g) of SKT occupying 1 g of the feces was measured in this way. Experimental results were represented by the mean values and the standard deviations thereof of the measurement values of the individuals from each test group, and the significance tests between the groups were performed using Student's t-test and Mann-Whitney U-test. The ratio of the number of individuals positive for SKT to the number of the individuals which composed each test group was calculated as the detection ratio. The measurement results are shown in FIG. 2.

As is evident from FIG. 2, the amount of SKT in the feces was significantly lower in the MGC group (13.1 ± 5.76 µg/g in the control group versus 7.3 ± 4.93 µg/g in the MGC group).

### [Organic acids in feces]

The feces 30 days after the weaning was diluted to 4 times with 0.03 M phosphate buffer (pH 7.4), and then centrifuged at 6,000 rpm for 15 minutes. The supernatant was filtrated through the filter of 0.45 µL (brand name: Minisart supplied from Sartorius), and then applied to HPLC.

### (HPLC condition)

Shimadzu Corporation LC-10 series
Column: Shin-pack SCR-102H, two columns
Column temperature: 40 °C
Mobile phase: Aqueous solution of 5 mM p-toluenesulfonic acid
Extraction phase: Aqueous solution of 5 mM p-toluenesulfonic acid, 20 mM Bis-Tris containing 100 µM toluenediaminetetraacetic acid
Flow rate: 0.8 mL/minute
Detector: Electric conductivity detector CDD-6A (Shimadzu Corporation)
Analysis time period: 60 minutes

Experimental results were represented by the mean values and the standard deviations thereof of the measurement values of the individuals from each test group, and the significance tests between the groups were performed using Student's t-test and Mann-Whitney U-test. The measurement results are shown in FIG. 3.

As is evident from FIG. 3, the levels of acetic acid, propionic acid and butyric acid were significantly higher in the MGC group than in the control group (acetic acid: 50.7 ± 9.27 mM in the control group versus 63.1 ± 5.55 mM in the MGC group; propionic acid: 26.4 ± 4.02 mM in the control group versus 32.1 ± 6.53 mM in the MGC group; butyric acid: 8.6 ± 3.03 mM in the control group versus 13.1 ± 4.16 mM in the MGC group). This way, the levels of acetic acid, propionic acid and butyric acid in the organic acids in the feces were significantly high. Thus, it is conceivable that the synbiotic effect was exerted by ingesting the present composition containing cellooligosaccharide and the Miyairi bacterial strain in combination.

### [Ammonia in feces]

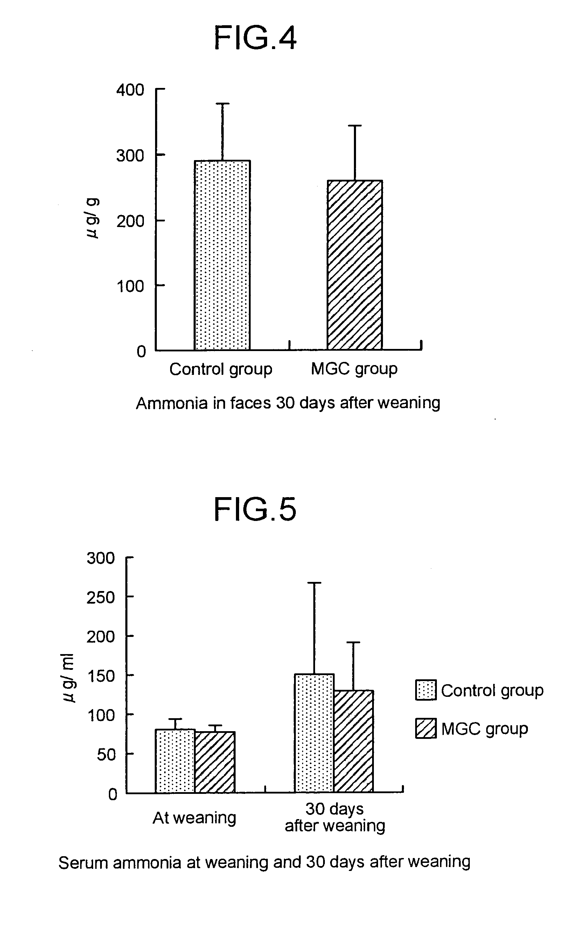

The feces 30 days after the weaning were collected in a tube for biochemical tests, and the level of ammonia was measured using Ammonia Test Wako. Experimental results were represented by the mean values of the measurement values of the individuals which composed each test group. The measurement results are shown in FIG. 4.

### [Ammonia in serum]

Ammonia produced by the enteric bacteria in the intestine is partially absorbed in the intestine and transferred to the blood. That is, the decrease of ammonia levels in the serum means that the ammonia-producing enteric bacteria were decreased and the ammonia amount in the intestine was decreased, indicating that the enteric environment was improved. Meanwhile, the increase of ammonia levels in the serum means that the ammonia-producing enteric bacteria were increased and the ammonia amount in the intestine was increased, indicating that the enteric environment was deteriorated.
The levels of ammonia in the serum, which can be referred to as an indicator of the change in the enteric environment, were measured as to the piglets at weaning and 30 days after the weaning. That is, a blood sample was collected from each individual, and proteins in the blood sample were eliminated using sodium tungstate. The resulting supernatant was collected in a tube for biochemical tests, and the level of ammonia was measured using Ammonia Test Wako. Experimental results were represented by the mean values of the measurement values of the individuals from each test group. The measurement results are shown in FIG. 5.

As is evident from FIG. 4, there was found a tendency that the ammonia concentration in the feces in the MGC group was lower than that in the control group. It was thus found that the enteric environment was improved and a bad smell of the feces was inhibited by the combination of cellooligosaccharide and the butyric acid bacteria.
As is evident from FIG. 5, there was also found a tendency that the ammonia concentration in the serum in the MGC group was lower than that in the control group. From this, it has been found that the ammonia amount in the intestine is decreased to improve the enteric environment by the combination of cellooligosaccharide and the butyric acid bacteria.

### [Example 4]

The efficacy of the present invention for domestic fowls was evaluated for the following parameters.
Two groups of neonatal chicks of broilers each having 20,000 individuals were prepared, as a control group and a present composition-administered group (test group).

A feedstuff was prepared by adding the present composition at 0.2% by weight to a commercially available feedstuff (no antibiotics added) for the broilers, wherein the present composition contains spores of the Miyairi bacterial strain (*Clostridium butyricum* MIYAIRI 588 (FERM BP-2789)) at 1×10⁸ CFU/g and 20% cellooligosaccharide (cellobiose 96% by weight, glucose 2% by weight, cellotriose 2% by weight). The feedstuff was administered to the individuals in the test group. Meanwhile, only the commercially available feedstuff (no antibiotics added) for the broilers was added to the individuals in the control group (no addition of the present composition).
When the individuals reached 18 days of age and 42 days of age, five chicks in each group were randomly selected and sacrificed, and an intestinal (appendix) content was collected. At the same time, five specimens of fresh feces in a chicken house of each group were collected.

### [Feeding test]

A weight gain (average body weight in each group), a feedstuff requirement (amount of the feedstuff required for weight gain of 1 kg) and a commercialization rate (rate of individuals which were not discarded (%)) were examined for the individuals in each group. The results are shown in Table 5.

**Table 5**

| | Control | Present composition |
|---|---|---|
| Average body weight (kg) | 2.59 | 2.75 |
| Feedstuff requirement (%) | 2.09 | 2.07 |
| Commercialization ratio (%) | 92.4 | 96.8 |

As is evident from Table 5, all of the average body weight, the feedstuff requirement, the commercialization rate and a discarded rate in the group using the present composition were improved when compared with the control group.

### [Organic acids in intestine]

The appendix content was diluted to 4 times with 0.03 M phosphate buffer (pH 7.4), and then centrifuged at 10,000 rpm for 15 minutes. The supernatant was filtrated through the filter of 0.45 µL (brand name: Minisart supplied from Sartorius), and then applied to HPLC. The HPLC condition was as follows.

### (HPLC condition)

Shimadzu Corporation LC-10 series
Column: Shin-pack SCR-102H, two columns
Column temperature: 40°C
Mobile phase: Aqueous solution of 5 mM p-toluenesulfonic acid
Extraction phase: Aqueous solution of 5 mM p-toluenesulfonic acid, 20 mM Bis-Tris containing 100 µM toluenediaminetetraacetic acid
Flow rate: 0.8 mL/minute
Detector: Electric conductivity detector CDD-6A (Shimadzu Corporation)
Analysis time period: 60 minutes

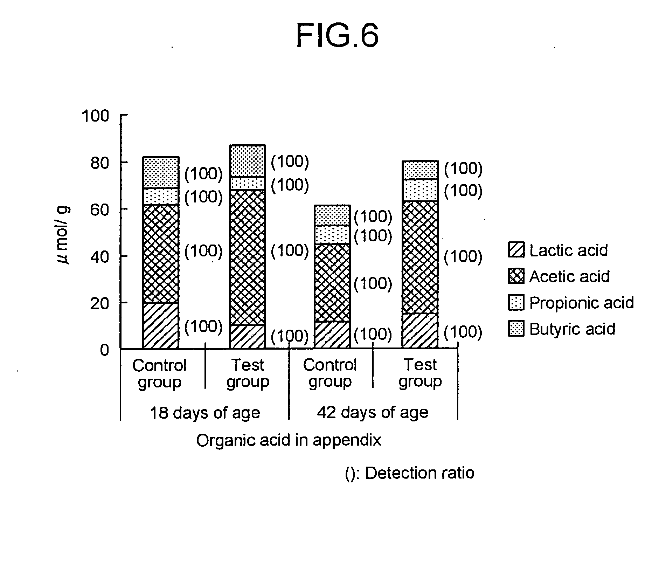

Experimental results were represented by the mean values and the standard deviations thereof of the measurement values of the individuals from each test group, and the significance tests between the groups were performed using Student's t-test and Mann-Whitney U-test. The measurement results are shown in FIG. 6.

As a result, as is evident from FIG. 6, acetic acid was the most abundant among the organic acids in the intestine. The detection ratios of propionic acid and butyric acid in the individuals which composed each group were 100%, and the detection ratios of the other organic acids were also 100%. In the appendix contents collected from the individuals at 18 days of age and 42 days of age, the ratios of acetic acid, propionic acid and butyric acid tends to be higher in the test group than in the control group, and this tendency was remarkable as to the amounts of acetic acid (amounts of acetic acid at 18 days of age: 42.8 ± 10.18 µmol in the control group versus 57.2 ± 7.66 µmol in the test group; amounts of acetic acid at 42 days of age: 32.4 ± 11.62 µmol in the control group versus 46.7 ± 3.82 µmol in the test group). Short chain fatty acids such as acetic acid, propionic acid and butyric acid are involved in growth inhibition of harmful bacteria by reduction of enteric pH. Those short chain fatty acids are also utilized as energy sources, and promote an intestinal peristaltic motion. Therefore, the fact that the amounts of the short chain fatty acids were increased in the test group than in the control group indicates that the enteric environment was improved by the combination of cellooligosaccharide and the butyric acid bacteria.

## Claims

1. A composition for a food or beverage comprising a butyric acid bacterium and cellooligosaccharide, said composition having a function to improve an enteric environment and/or a function to protect against an infectious disease.

2. The composition for the food or beverage according to claim 1 wherein the butyric acid bacterium is *Clostridium butyricum.*

3. A feedstuff comprising a butyric acid bacterium and cellooligosaccharide, said feedstuff having a function to improve an enteric environment and/or a function to protect against an infectious disease.

4. A food comprising a butyric acid bacterium and cellooligosaccharide, said food having a function to improve an enteric environment and/or a function to protect against an infectious disease.

5. Use of a butyric acid bacterium and cellooligosaccharide as a composition for a food or beverage having a function to improve an enteric environment and/or a function to protect against an infectious disease.

6. Use of a butyric acid bacterium and cellooligosaccharide as a feedstuff or a food having a function to improve an enteric environment and/or a function to protect against an infectious disease.
